# EUROPEAN PATENT APPLICATION

(11) **EP 0 897 720 A1**
(43) Date of publication of application: **24.02.1999**
(21) Application number: 98306062.5
(22) Date of filing: 30.07.1998
(51) Int. Cl.: A61K 31/135

(54) **Droloxifene for prevention of breast cancer**

(30) Priority: 13.08.1997 US 55552 P
(71) Applicant: Pfizer Products Inc., Groton, CT 06340-5146 (US)
(72) Inventor: Kraska, Allen Richard, Old Lyme, Connecticut 06371 (US)
(74) Representative: Wood, David John

(57) **Abstract**

Use of droloxifene for the manufacture of a medicament for the prevention of breast cancer.

## Description

### Field of the Invention

This invention relates to remedies for preventing breast cancer comprising, as active ingredient, droloxifene having the chemical structure represented by the following formula, 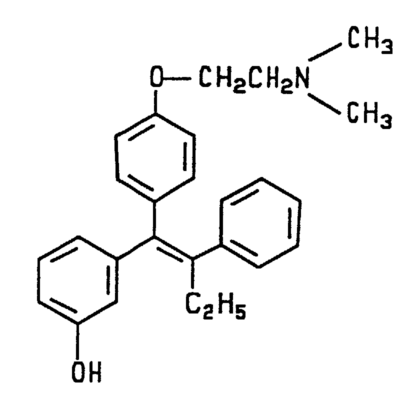 or a pharmaceutically acceptable salt thereof.

### Background of the Invention

Droloxifene is a known compound disclosed in United States Patent 5,047,431 in which it is disclosed as an anti-tumor agent, particularly for treatment of cancer of the breast Droloxifene is also useful for the relief of bone diseases caused by the deficiency of estrogen or the like, which are often observed in women after menopause or those with the ovaries removed. U.S. Patent No. 5,254,594.

Richardson, U.S. Patent No. 4,198,435 issued April 15, 1980, claims the use of trans-4-hydroxtamoxifen as an antiestrogen; Richardson, U.S. Patent No. 4,206,234, issued June 3, 1980, claims cis-4-hydroxytamoxifen and its use as an antiestrogen; Harper et al., U.S. Patent No. 4,536,516, issued August 20, 1985, claims tamoxifen and its salts and their use in the treatment of hormone dependent tumors; Richardson, U.S. Patent No. 4,623,660, issued November 18, 1986, claims derivatives of cis-and trans-4-hydroxytamoxifen and their use as antiestrogens.

Tamoxifen is a known antiestrogenic compound which is useful for the treatment and prevention of mammalian breast carcinoma; see The Merck Index, 11th Ed., 1430 (1989), and U.S. Patent No. 5,604,248.

Raloxifine, 6-hydroxy-2-(4-hydroxyphenyl)-3-[4-(2-pyperidinoethoxy) benzoyl] benzol[b] thiophen is claimed in U.S. Patent No. 4,418,068 in a method for treating mammary cancer. The use of Raloxifene to minimize the uterotrophic effect of Tamoxifen in treatment and prevention of breast cancer is disclosed in U.S. Patent No. 5,604,248. Raloxifine is claimed for treatment of benign breast disorders in U.S. Patent No. 5,593,987.

### Summary of the Invention

This invention provides a method for preventing breast cancer in mammals which comprises administering to said mammal an amount of droloxifene or a pharmaceutically acceptable salt thereof which is effective in preventing breast cancer.

### Detailed Description of the Invention

The preparation of droloxifene (1-[4'-(2-dimethylaminoethoxy)phenyl]-1-(3'-hydroxyphenyl)-2-phenylbut-1-ene) and pharmaceutically acceptable salts thereof is described in United States Patent No. 5,047,431 which is incorporated herein by reference.

As used in this application, "prevention" means avoidance of the appearance of occult disease. "Treating" means curing, or alleviating the symptoms of a disease or condition.

The remedies for the prevention of breast cancer of this invention comprise, as active ingredient, droloxifene or a salt thereof. The pharmaceutically acceptable salts of droloxifene are salts of non-toxic types commonly used, such as salts with organic acids (e.g., formic, acetic, trifluoroacetic, citric, maleic, tartaric, methanesulfonic, benzenesulfonic or toluenesulfonic acids), inorganic acids (e.g. hydrochloric, hydrobromic, sulfuric or phosphoric acids), and amino acids (e.g., aspartic or glutamic acids). These salts may be prepared by the methods known to chemists of ordinary skill.

The remedies for prevention of breast cancer of this invention may be administered to animals including humans orally or parenterally in the conventional form of preparations, such as capsules, microcapsules, tablets, granules, powder, troches, pills, suppositories, injections, suspensions and syrups.

The remedies for prevention of breast cancer of this invention can be prepared by the methods commonly employed using conventional, organic or inorganic additives, such as an excipient (e.g., sucrose, starch, mannitol, sorbitol, lactose, glucose, cellulose, talc, calcium phosphate or calcium carbonate), a binder (e.g., cellulose, methylcellulose, hydroxymethylcellulose, polypropylpyrrolidone, polyvinylprrolidone, gelatin, gum arabic, polyethyleneglycol, sucrose or starch), a disintegrator (e.g., starch, carboxymethylcellulose, hydroxypropylstarch, low substituted hydroxypropylcellulose, sodium bicarbonate, calcium phosphate or calcium citrate), a lubricant (e.g., magnesium stearate, light anhydrous silicic acid, talc or sodium lauryl sulfate), a flavoring agent (e.g., citric acid, menthol, glycine or orange powder), a preservative (e.g., sodium benzoate, sodium bisulfite, methylparaben or propylparaben), a stabilizer (e.g., citric acid, sodium citrate or acetic acid), a suspending agent (e.g., methylcellulose, polyvinylpyrrolidone or aluminum stearate), a dispersing agent (e.g., hydroxypropylmethylcellulose), a diluent (e.g., water), and base wax (e.g., cocoa butter, white petrolatum or polyethylene glycol). The amount of the active ingredient in the medical composition may be at a level that will exercise the desired therapeutical effect; for example, about 1 mg to 100 mg in unit dosage for both oral and parenteral administration.

The active ingredient may be usually administered once to four times a day with a unit dosage of 0.25 mg to 100 mg in human patients, but the above dosage may be properly varied depending on the age, body weight and medical condition of the patient and the type of administration. One dose per day is preferred.

The following Examples will serve to illustrate, but do not limit the invention which is defined by the claims.

### EXAMPLE 1

| Droloxifene Citrate Tablets | |
|---|---|
| Droloxifene citrate | 100 g |
| Lactose | 1190 g |
| Low substituted hydroxypropylcellulose | 250 g |
| Polyvinylpyrrolidone | 50 g |
| Magnesium stearate | 10 g |

The components listed above are mixed together by the usual method, and the mixture thus obtained is compressed into 10,000 tablets each containing 10 mg of droloxifene citrate.

### EXAMPLE 2

### Phamacological Tests

### a) Binding Affinity to the Estradiol Receptor

The binding affinity to the estradiol receptor was measured according to the method of N. Devleeschouwer, G. Leclercq, A. Danguy and J. C. Heuson [Europ. J. Cancer, 14, 721-723 (1978)]. The uterus cytosol of female, prepuberal white rabbits of 2 kg. weight (New Zealand rabbits) was incubated for 18 hours at 4° C. with 2.5 X 10 -⁹M[³H)-estradiol as well as with the addition of unlabelled estradiol (control) or test substance of different concentration. The binding affinity to the estradiol receptor is expressed by the concentration of unlabelled estradiol (control) or test substance which is added to the uterus cytosol and brings about a 50% replacement of the [³H]-estradiol bound to the estradiol receptor.

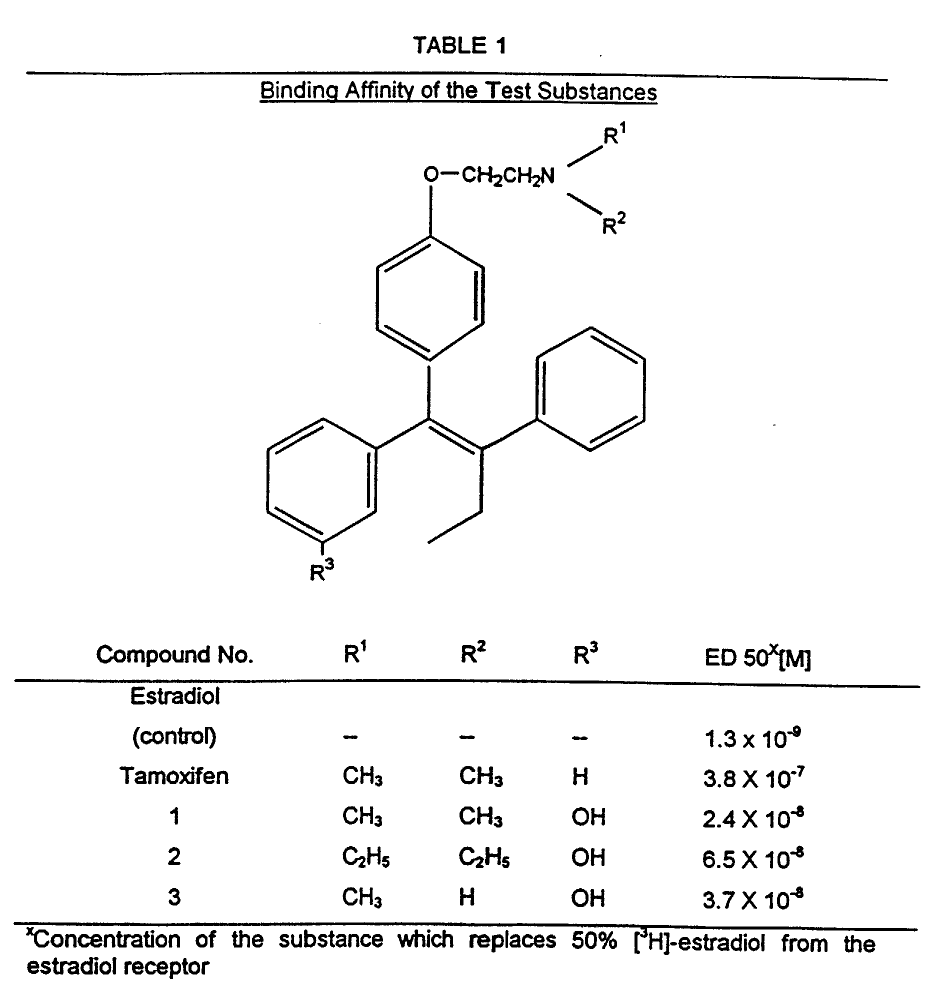

### b) Anti-uterotrophic Effect

The anti-uterotrophic effect was determined according to a modified "Dorfman Test" [R. I. Dorfman, Methods in Hormone Research II, p. 707, Academic Press, New York - London, 1962]on puberal, female sprague-Dawley rats.

The test compounds were put in 0.25% aqueous agar suspension and administered by stomach tube six times weekly over a period of 21 days. At the end of testing, the uterine weight of the animals reacted with active ingredient was related to the uterine weight of the control animals which only received a blank agar suspension.

**TABLE 2**

| Anti-uterotrophic Activity of the Test Substances | | | |
|---|---|---|---|
| Compound No. | No. of Test Animals | Dose mg./kg./ day | Uterine Weight compared to Controlled Animals |
| Tamoxifen | 10 | 3 | -40% |
| 1 | 10 | 3 | -42% |
| 2 | 10 | 3 | -39% |
| 3 | 10 | 3 | -53% |

### c) Inhibitory Effect on the Mammary Tumor

The inhibitory effect on the tumor was determined on the model of the 7,12-dimethylbenz(a)anthraceneinduced mammary tumor of the female Sprague-Dawley rat (Hanover breed) according to the method of M.J. Golder [Europ. J. Cancer 11,571 (1975)]and D. P. Griswold et al. [Cancer Research 26, 2169 (1966)].

The test compounds were put in 0.25% agar suspension and administered by stomach tube six times weekly over a period of 28 days. Twice weekly and on the 28th day of testing the number of animals was determined and the tumor surface (mm²/animal) of the therapy animals and control animals was measured. At the end of testing, the percentage increase of the average tumor surface of the treated animals was determined in comparison to the control animals, the surface of the latter being taken as 100%.

**TABLE 3**

| Anti-uterotrophic Activity of the Test Substances | | | |
|---|---|---|---|
| Compound No | No. of Test Animals | Dose mg./kg./ Day | Relative Increase of the Average Tumor Surface |
| Blank Control | 10 | - | 100% |
| Tamoxifen | 12 | 3 | 35% |
| 1 | 12 | 3 | 23% |

### EXAMPLE 3

Twelve thousand women are selected for the clinical study. The women are at enhanced risk of breast cancer because of age (over 60 years) or a family history of breast cancer, but otherwise are in good general health. The study has a placebo control group, i.e., the women are divided into two groups, one of which receives a compound of droloxifene as the active agent and the other receives a placebo. Women in the test group receive between 20 - 80 mg of the drug per day by the oral route. They continue this therapy for three years. Each patient is examined for breast cancer by mammography every twelve months. A smaller number of newly developed breast cancer in the group receiving droloxifene indicates that droloxifene prevents breast cancer.

It is believed that droloxifene (compound 1) is relatively quite free of histological side-effect common to earlier anti-estrogens. Tamoxifen, for example, causes abnormal growth of uterine epithelial cells. Preliminary studies indicate that the present compounds either cause no abnormality of epithelial cells of the uterus, or cause only extremely slight abnormal growth.

The exact mechanism of an antiestrogen in treating or preventing mammary cancer is not known. It is believed, however, that the antiestrogen, circulating in the body, competes with enogenous estrogens for estrogen receptor sites in the cancer. To be effective, the antiestrogen must find and bind to receptor sites, and prevent estradiol from occupying them. Clearly, if the antiestrogen has inherent estrogenicity of its own, it is likely to enable the cancer to grow just as if the cancer had absorbed endogenous estrogen.

The biological tests reported above show the low estrogenicity, high antiestrogenicity and strong affinity for estrogen receptor sites of droloxifene. These factors, as explained in U.S. Patent No. 4,418,068, predict that a compound which shows these properties will be effective in alleviating mammary cancers in patients suffering from or at risk of such a cancer, Col. 37, lines 59-65.

Accordingly, a most important embodiment of the present invention is a method of preventing mammary cancers which comprises administering droloxifene in an effective amount to a patient at risk of such a cancer.

## Claims

1. The use of droloxifene or of a pharmaceutically acceptable salt thereof for the manufacture of a medicament for preventing breast cancer.

2. The use of claim 1 wherein the salt is the citrate salt.
